# FASCICULE DE BREVET EUROPEEN

(11) **EP 2 651 866 B1**
(45) Date de publication et mention de la délivrance du brevet: **28.11.2018**
(21) Numéro de dépôt: 11817382.2
(22) Date de dépôt: 16.12.2011
(51) Int. Cl.: C07C 41/16, C07C 41/01

(54) **PROCEDE DE PREPARATION D'ETHER DE POLYOL**
VERFAHREN ZUR HERSTELLUNG EINES POLYOLETHERS
PROCESS FOR PREPARING A POLYOL ETHER

(30) Priorité: 17.12.2010 FR 1060745
(43) Date de publication de la demande: 23.10.2013
(73) Titulaire: Fonds de Developpement des Filières des Oleagineux et Proteagineux FIDOP, 75008 Paris (FR); Université Claude Bernard Lyon I, 69622 Villeurbanne Cedex (FR); Centre National de la Recherche Scientifique, 75016 Paris (FR)
(72) Inventeur: LEMAIRE, Marc, F-69100 Villeurbanne (FR); DAYOUB, Wissam, F-69100 Villeurbanne (FR); SUTTER, Marc, F-69100 Villeurbanne (FR); RAOUL, Yann, F-02650 Crezancy (FR)
(74) Mandataire: Hubert, Philippe
(86) Numéro de dépôt international: PCT/FR2011/053039
(87) Numéro de publication internationale: WO 2012/080682

(56) Documents cités:
- EP-A1- 0 624 563
- US-A1- 2006 128 979
- US-B1- 6 218 580
- SHI Y ET AL: "Straightforward selective synthesis of linear 1-O-alkyl glycerol and di-glycerol monoethers", TETRAHEDRON LETTERS, ELSEVIER, AMSTERDAM, NL, vol. 50, no. 49, 9 décembre 2009 (2009-12-09), pages 6891-6893, XP026699729, ISSN: 0040-4039, DOI: DOI:10.1016/J.TETLET.2009.09.134 [extrait le 2009-09-27]
- SHI YAN AND CO: "One-step selective synthesis of branched 1-O-alkyl-glycerol/diglycerol monoethers by catalytic reductive alkylation of ketones", SCIENCE CHINA CHEMISTRY, vol. 53, no. 9, 1 septembre 2010 (2010-09-01), pages 1953-1956, XP002649430,
- AVAEV V I AND CO: "Effect of the nature of the carrier and reduction conditions on the properties of rhenium catalysts of hydrogenation of ethyl acetate", RUSSIAN CHEMICAL BULLETIN, vol. 37, no. 1, 1 janvier 1988 (1988-01-01), pages 15-19, XP002649434,
- ZHIBIAO MAO ET AL: "Catalytic hydrosilation of organic esters using manganese carbonyl acetyl complexes, (L)(CO)4MnC(O)CH3 (L = CO, PPh3)", JOURNAL OF THE AMERICAN CHEMICAL SOCIETY, AMERICAN CHEMICAL SOCIETY, WASHINGTON, DC; US, vol. 117, no. 40, 1 janvier 1995 (1995-01-01), pages 10139-10140, XP002270419, ISSN: 0002-7863, DOI: DOI:10.1021/JA00145A036

## Description

La présente invention a pour objet un procédé de préparation d'éther de polyol à partir d'un polyol et d'un acide carboxylique ou d'un ester d'acide carboxylique.

Ce procédé permet, entre autre, de réaliser la synthèse directe et sélective de monoalkyléthers de polyols à partir d'un polyol et d'un acide carboxylique ou d'un ester d'acide carboxylique en conditions douces.

### ETAT DE LA TECHNIQUE

La synthèse d'éthers symétriques ou non est généralement réalisée selon la réaction de Williamson bien connue de l'homme du métier comme étant l'une des plus anciennes réactions organiques industriellement utilisée pour effectuer ce type de transformation. Cette voie de synthèse consiste en la réaction d'un ion alcoolate, formé par déprotonation de l'alcool correspondant par une base, avec un dérivé halogéné pour former un éther.

Cette réaction présente néanmoins de multiples inconvénients du faite qu'elle nécessite l'utilisation de bases fortes telles que l'hydrure de sodium, l'hydroxyde de sodium ou de potassium, la rendant inappropriée vis-à-vis des molécules sensibles aux bases.

D'autres procédés d'alkylation faisant réagir des molécules très réactives telles que les aldéhydes ou des cétones avec des polyols pour obtenir des polyols alkylés en présence de catalyseurs et d'hydrogène ont également été décrits.

On citera notamment la demande de brevet internationale WO 2010/027663 A1 et le brevet US 5,446,210 qui décrivent de façon générale la synthèse d'éthers de polyols à partir d'un aldéhyde ou d'une cétone et d'un polyol par voie d'une alkylation catalytique réductrice.

L'un des inconvénients majeurs de ces procédés d'alkylation réside essentiellement dans la difficulté d'obtention, de l'instabilité et du coût généralement élevé des aldéhydes et cétones de départ.

Le document (Tetrahedron Letters, 2009, 50, 6891-6893) décrit la préparation de monoéthers de 1-O-alkyl glycérol et diglycérol obtenus par alkylation réductrice d'aldéhydes linéaires en présence d'un catalyseur Pd/C, de dihydrogène sous pression et d'un acide de Brønsted comme co-catalyseur.

Le document (Science China Chemistry, 2010, 53, 1953-1956) décrit la préparation de monoéthers de 1-O-alkyl glycérol et diglycérol obtenus par alkylation réductrice de cétones linéaires en présence d'un catalyseur Pd/C, de dihydrogène sous pression et d'un acide de Brønsted comme co-catalyseur.

Aucun de ces documents ne décrit ni ne suggère une voie de synthèse et des conditions opératoires susceptibles de permettre l'obtention d'éthers de polyols à partir d'un acide carboxylique ou d'un ester d'acide carboxylique.

### DESCRIPTION DES FIGURES

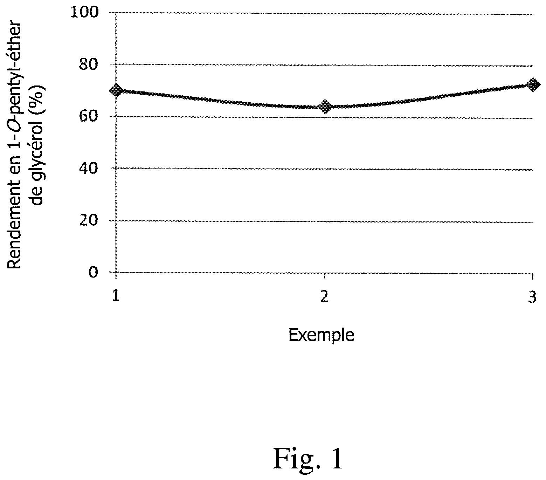

La Figure 1 représente l'évolution du rendement en 1-O-pentyl-éther de glycérol après recyclage du système catalytique pour les exemples 30, 31 et 32 (1, 2 et 3 respectivement).

### DESCRIPTION DE L'INVENTION

La présente invention a pour but général de fournir une nouvelle voie d'accès aux molécules dérivées de polyols, notamment les polyols alkylés et préférentiellement les polyols monoalkylés de façon sélective.

Un objet de la présente invention a également pour but de proposer une nouvelle réaction de O-alkylation à partir de réactifs de départ stables et facilement accessibles commercialement ou par voie de synthèse tels que les acides carboxyliques ou les esters d'acides carboxyliques.

Un autre objet de la présente invention est de fournir une réaction pouvant être mis en oeuvre dans des conditions douces c'est-à-dire compatibles, par exemple, avec des groupes fonctionnels sensibles aux bases, et de manière aisée avec des réactifs de départ qui ne requièrent pas de manipulations particulières (par exemple, due à leur instabilité vis-à-vis de l'oxydation) et à un coût réduit avec des réactifs de départ qui sont généralement facile d'accès.

Ainsi, selon un premier aspect, la présente demande concerne un procédé de préparation en une seule étape d'un éther de polyol de formule (I) caractérisé en ce qu'il comprend une étape d'alkylation réductrice faisant intervenir un composé de formule générale (II) et un composé de formule générale (III) : 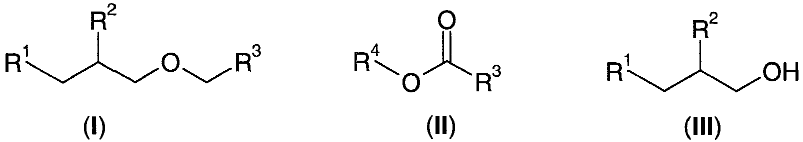 dans lesquelles :
R¹ et R² représentent indépendamment l'un de l'autre des groupes choisis parmi un atome d'hydrogène, un groupement -OH, ou -(OCH₂-CH(OH)-CH₂)ₚ-OH avec 1≤p≤10,
R⁴ représente un atome d'hydrogène ou un groupe choisi parmi un groupe alkyle en C₁-C₅₀, un groupe alcènyle en C₂-C₅₀, un aryle, un cycloalkyle en C₃-C₈, un groupe -CH₂-CH[O-[C(=O)]ₙ-R₅]-CH₂-O-[C(=O)]ₘ-R₆, ledit groupe pouvant être non-substitué ou substitué ;
R³, R⁵ et R⁶ représentent indépendamment les uns des autres des groupes choisis parmi un alkyle en C₁-C₅₀, un alcènyle en C₂-C₅₀, un aryle, un cycloalkyle en C₃-C₈, lesdits groupes pouvant être non-substitués ou substitués ;
n et m sont indépendants l'un de l'autre et sont égaux à 0 ou 1 ;
R⁵ est un atome d'hydrogène lorsque n=0;
R⁶ est un atome d'hydrogène lorsque m=0 ;
et dans lesquelles au moins un de R¹ et R² est un groupe hydroxyle - OH, ladite étape d'alkylation réductrice étant réalisée :
- en présence d'un catalyseur métallique choisi parmi le palladium, le rhodium, le ruthénium, le cobalt, le platine, l'iridium ou le nickel, ledit catalyseur étant éventuellement associé à un support choisi parmi le charbon, l'alumine, la silice ou l'aluminosilicate ;
- en présence d'un catalyseur acide ; et
- en présence d'un gaz réducteur, de préférence le dihydrogène.

Il est important de noter qu'un homme de métier comprendrait immédiatement que le procédé selon l'invention a vocation à s'appliquer de façon générale à la réaction de toute molécule de formule générale (III) dès lors qu'elle possède un groupe hydroxyle -OH disponible à l'alkylation, ainsi qu'à toute molécule de formule générale (II) dès lors qu'elle puisse réagir avec un groupe hydroxyle -OH disponible à l'alkylation d'une molécule de formule générale (III).

La présente invention a donc pour principe général la synthèse d'un polyol dans lequel un groupe hydroxyle -OH, et préférentiellement dans lequel seulement un groupe hydroxyle -OH parmi plusieurs autres groupes hydroxyles -OH chimiquement équivalents ou non, est alkylé par un groupe fonctionnel provenant d'un acide carboxylique ou d'un ester d'acide carboxylique.

Selon un mode de réalisation avantageux, ladite étape d'alkylation réductrice sera réalisée en présence d'un catalyseur choisi parmi le palladium sur charbon, le ruthénium étain, Ru/C, Pd/Al₂O₃, Pd/SiO₂, en quantité inférieure à 5%, de préférence en quantité inférieure à 2%, et de préférence encore en quantité inférieure à 1 % molaire de catalyseur par rapport à la quantité de composé de formule générale (II).

Il est bien entendu possible d'utiliser dans le cadre de la présente invention tout catalyseur comprenant du palladium, du rhodium, du ruthénium, du cobalt, du platine, de l'iridium, du nickel ou un autre métal connu pour catalyser une réaction d'hydrogénation ledit catalyseur étant éventuellement associé à un support choisi parmi le charbon, l'alumine, la silice ou l'aluminosilicate.

Selon un autre mode de réalisation avantageux, cette étape d'hydrogénation est réalisée à une température inférieure à 200°C, de préférence inférieure à 180°C et encore plus préférentiellement inférieure à 150°C.

Selon un mode de réalisation particulièrement avantageux, cette étape d'alkylation réductrice est réalisée en présence d'un catalyseur acide (de Bronsted ou de Lewis) choisi parmi l'acide camphosulphonique, l'acide paratoluène sulfonique, les résines sulfoniques acides (type Amberlyst, Nafion), l'acide trifluoroacétique, la silice, la silice alumine, l'acide phosphorique supporté sur silice, BF₃.Et₂O ou les zéolithes, avantageusement en quantité inférieure à 10% en masse, de préférence inférieure à 5%, par rapport à la masse totale de composé de formule générale (II).

Les catalyseurs acides se présentant sur support solide, tels que les résines sulfoniques acides, sont particulièrement avantageux dans la mesure où ils peuvent être extraits du milieu réactionnel facilement après réaction par simple filtration pour leurs éventuelles réutilisations.

Selon un autre mode de réalisation particulièrement avantageux, le catalyseur acide est un catalyseur acide se présentant sur support solide formant, avec le catalyseur métallique précité, un système catalytique recyclable.

Le système catalytique, catalyseur métallique/catalyseur acide sur support solide (par exemple Pd/C / Amberlyst), employé dans le procédé selon l'invention peut être recyclé, c'est-à-dire utilisé plusieurs fois, à savoir au moins 3 fois, voire au moins 10 fois, voire même au moins 20 fois, sans perte significative, ni de rendement ni de sélectivité (en faveur de la formation d'un polyol de formule (I)). Habituellement ce système sera utilisé entre 3 et 10 fois.

Ceci permet notamment au procédé de l'invention d'être compatible avec un procédé industriel « en continu ».

Par « perte significative de rendement » on entend une variation de rendement d'au plus 50%, d'au plus 30%, d'au plus 20%, d'au plus 10%, d'au plus 5% et de préférence d'au plus 1% entre le rendement de la « nième » réaction (dernière réaction réalisée avec le système catalytique) et le rendement le plus élevé obtenu pour les réactions précédentes de rang 1 (première réaction réalisée avec le système catalytique) au rang n-1.

Par « perte significative de sélectivité » on entend une variation de sélectivité d'au plus 70%, d'au plus 50%, d'au plus 30%, d'au plus 20%, d'au plus 10%, d'au plus 5% et de préférence d'au plus 3% entre la sélectivité de la « nième » réaction (dernière réaction réalisée avec le système catalytique) et la sélectivité la plus élevée obtenue pour les réactions précédentes de rang 1 (première réaction réalisée avec le système catalytique) au rang n-1.

Avantageusement, lorsque le composé de formule générale (II) est un acide carboxylique l'introduction du catalyseur acide peut être évitée. Dans ce mode de réalisation, l'acide carboxylique de formule générale (II) peut agir en tant que catalyseur acide de la réaction.

Selon un autre mode de réalisation avantageux, l'étape d'hydrogénation est réalisée sous une pression en gaz réducteur, notamment le dihydrogène H₂, comprise entre 1 et 150 bars, de préférence entre 1 et 100 bars, plus préférentiellement entre 1 et 50 bars, encore plus préférentiellement entre 10 et 50 bars, et encore plus préférentiellement entre 30 et 50 bars.

L'un des avantages supplémentaires du procédé selon l'invention est qu'un composé de formule (I) peut être obtenu en une seule étape réactionnelle. Cela permet d'accéder à des polyols alkylés de structures plus ou moins complexes rapidement et à un coût réduit.

La présente invention permet également de valoriser les ressources mondiale en polyols naturels comme notamment le glycérol et ses dérivés,
en les utilisant en tant que matières premières par exemple dans l'industrie chimique. Un tel procédé peut donc être répertorié parmi les procédés de synthèse dits "propres" c'est-à-dire contribuant à la protection de l'environnement.

Bien que, tel que susmentionné, le procédé selon l'invention possède un champ d'application très général permettant la O-alkylation d'un polyol à partir d'un acide carboxylique ou d'un ester, les composé de départ précitées peuvent néanmoins être choisis de préférence parmi des composés de formule (II) dans laquelle :
R³ représente un groupe choisi parmi un alkyle en C₁-C₅₀ et un alcènyle en C₂-C₅₀ et R⁴ représente un groupe choisi parmi un atome d'hydrogène et un groupe alkyle en C₁-C₅.

Plus préférentiellement, dans les composés de formules (II) et (III) précitées :
R¹ représente un groupe choisi parmi un atome d'hydrogène, un groupement -OH, -(OCH₂-CH(OH)-CH₂)ₚ-OH avec 1≤p≤10;
R² représente un groupe hydroxyle -OH ;
R³ représente un groupe choisi parmi un alkyle en C₁-C₂₈, de préférence en C₁-C₂₄, un alcènyle en C₂-C₂₈, de préférence en C₂-C₂₄.

Encore plus préférentiellement, dans la formule (III) précitée, R¹ et R² représentent tous les deux un groupe hydroxyle -OH.

À titre d'exemple de composés de formule générale (III) pouvant être utilisés dans le procédé selon l'invention on peut citer le glycérol, les dérivés du glycérol et autres substrats polyhydroxylés tels que 1,2-éthanediol, 1,2-propanediol, 1,3-propanediol, 1,4-butanediol, pentaérythritol, triméthylolpropane, les polyglycérols, les polyoxyéthylènes, les polyoxypropylènes.

Le composé de formule générale (II), quant à lui, peut être plus préférentiellement choisi parmi l'acide valérique, l'acide caproïque, l'acide adipique, l'acide pélargonique, l'acide azélaïque, l'acide brassylique, l'acide myristique, l'acide stéarique, l'acide oléique, l'acide 9Z-octadécènedioïque, l'acide érucique et les esters de méthyle correspondants.

Sans limitation particulière, les composés de formule générale (II) pourront également être choisis parmi les acides gras, les esters gras ou les triglycérides ou toutes autres substances naturelles ou dérivés de substances naturelles ayant au moins une fonction acide carboxylique ou ester.

Parmi ces substances naturelles ou dérivées de substances naturelles on peut citer l'huile de soja, huile de tournesol, huile de colza, huile de lin, huile d'olive, huile de ricin, huile d'arachide, huile de palme, etc.

La présente invention n'est bien évidemment pas limitée au type d'appareillage de chauffage, de pressurisation et/ou d'agitation utilisé dans le procédé.

Le procédé peut également s'effectuer à l'aide de tous types d'appareils à micro-ondes pouvant fournir la température et la pression nécessaire à la réalisation du procédé selon l'invention.

En règle générale on utilisera un composé de formule (II) et un composé de formule (III) dans des proportions molaires comprises entre 1/1 et 1/50 et préférentiellement comprises entre 1/2 et 1/10.

Les rendements isolés sont avantageusement supérieurs à 10%, de préférence supérieurs à 15%, de préférence encore supérieurs à 20%, de préférence compris entre 22% et 71% et plus préférentiellement compris entre 22% et 90%.

Dans le cadre de la présente description, par "rendement isolé", on entend le rendement massique calculé à partir du produit de formule générale (I) isolé/purifié du milieu réactionnel brut par une ou plusieurs méthode(s) de purification connues de l'homme du métier.

Dans le cadre de la présente description, par "alkyle", on entend une chaine hydrocarbonée saturée linéaire ou ramifiée de formule chimique CₙH₂ₙ₊₁. En tant qu'exemple de groupe alkyle on peut citer les groupes méthyle, éthyle, n-propyle, isopropyle, n-butyle, isobutyle, sec-butyle, t-butyle, pentyle, capryle, lauryle, myristyle, palmityle, stéaryle, arachidyl, lignocéryle, etc.

Dans le cadre de la présente description, par "alcènyle", on entend une chaine hydrocarbonée linéaire ou ramifiée comportant au moins une insaturation.

Dans le cadre de la présente description, par "aryle", on entend un groupe monocyclique ou polycyclique ayant un système d'électrons π délocalisé de type (4n+2) dans lequel n est un nombre entier, incluant les cycles ne contenant que des atomes de carbones, mais aussi les cycles contenant au moins un hétéroatome choisis parmi N, O et S (c'est-à-dire des groupes hétéroaryles).

En tant qu'exemple de groupe aryle on peut citer notamment les groupes phényle, naphtyle, pyrridyle, furyle, furanyle, thiényle, pyrrolyle indolyle, imidazolyle, thiazolyle, pyrrolidyle, pyrimidyle.

Dans le cadre de la présente description, par "aryloxy", on entend un groupe aryle lié à un atome d'oxygène.

Dans le cadre de la présente description, par "cycloalkyle", on entend un groupe hydrocarboné saturé monocyclique ou polycyclique, de préférence contenant de 1 à 3 cycles et de 3 à 7 atomes de carbone par cycle pouvant être également accolé à un cycle carbocyclique insaturé en C₃-C₇. Parmi les groupes cycloalkyle on peut citer, par exemple, les groupes cyclopropyle, cyclobutyle, cyclopentyle, cyclohexyle, cycloheptyle, cycloctyle, cyclodecyle, cyclododecyle, adamantyle, etc. Le terme "cycloalkyle" comprend également les groupes cyclique substitué ou non contenant au moins un hétéroatome choisis parmi N, O et S (c'est-à-dire des groupes hétérocycliques).

Dans le cadre de la présente description, par "cycloalkoxy", on entend un groupe cycloalkyle lié à un atome d'oxygène.

Les groupes alkyle, alcènyle, aryle, aryloxy, cycloalkyle et cycloalkoxy dont la définition vient d'être rappelée peuvent être substitués par un ou plusieurs substituents généralement choisis parmi les atomes d'halogène et les groupes hydroxyle, alkoxy, oxo, alkanoyle, aryloxy, alkanoyloxy, amino, alkylamino, arylamino, arylalkylamino, alkanoylamino, aroylamino, aralkanoylamino, thiol, alkylthio, arylthio, arylalkylthio, alkylthiono, arylthiono, arylalkylthiono, alkylsulfonyle, arylsulfonyle, arylalkylsulfonyle, sulfonamido, nitro, cyano, carboxy, carbamyle, alkoxycarbonyle, aryle, guanidino, hétérocyclyle.

Dans le cadre de la présente description, par "O-alkylation", on entend la formation d'une liaison covalente entre un atome d'oxygène d'un substrat et un groupe alkyle ou un dérivé d'un groupe alkyle.

Dans le cadre de la présente description, par "polyol", on entend un composé organique contenant au moins deux groupes hydroxyles -OH tels que, par exemple, les (1,n)-diols, les glycérols et leurs dérivés.

Dans le cadre de la présente description, par "acide carboxylique", on entend toute substance organique possédant au moins une fonction carboxyle -COOH.

Les modes de réalisations et conditions réactionnelles préférés et envisagés dans le cadre de la présente invention sont particulièrement ceux correspondants aux combinaisons, selon l'invention, de chacune des alternatives d'une des listes précitées avec chacune des alternatives des autres listes précitées.

Par conséquent, chaque combinaison d'éléments issue des listes d'alternatives précitées relatives au choix :
- du catalyseur et de la quantité de catalyseur,
- du catalyseur acide et de la quantité de catalyseur acide,
- de la pression de dihydrogène H₂,
- de la température de réaction,
- de la structure chimique et de la quantité du composé (II),
- de la structure chimique et de la quantité du composé (III),
est particulièrement envisagée dans le cadre de la présente invention.

Les produits obtenus par un procédé selon l'invention peuvent être utilisés dans divers secteurs d'application, notamment en tant que stimulants pour la formation de cellules sanguines dans la moelle osseuse, en tant qu'agent anti-inflammatoires et anti-tumorales, en tant qu'additif dans la préparation de formulations anti-transpirantes, en tant qu'additif pour les formulations destinées au soin de la peau, en tant qu'additif dans les formulations déodorantes, dans la formulation de compositions nettoyantes pour l'hygiène corporelle ou bien encore en tant qu'émulsifiants.

L'invention sera maintenant illustrée par les exemples non limitatifs suivants.

### EXPÉRIMENTATIONS RÉALISÉES

Les acides ou esters ont été fournis par Acros, Sigma-Aldrich. Les réactifs de départ, catalyseurs, co-catalyseurs acides, ont été fournis par Strem, Acros, Sigma-Aldrich. Tous les réactifs ont été utilisés sans purification supplémentaire. Les mesures de masse exacte ont été réalisées avec une GC/MS Thermo DSQ. équipée d'une colonne de type DB5 (0.15 mm, 40 m), en utilisant la méthode suivante : (Paramètres donnés en langue anglaise tels qu'utilisés par l'homme du métier utilisant ce type d'appareillage)

### Oven Method

Initial Temperature (C): 70
Initial Time (min): 2.00
Number of Ramps: 1
Rate #1 (deg/min): 15.0
Final Temperature #1 (C): 330
Hold Time #1 (min): 15.00
Post Run Temperature: Off
Enable Cryogenics: Off
Maximum Temperature (C): 350
Prep Run Timeout (min): 10.00
Equilibration Time (min): 0.50

### Right SSL Method

Base Temperature: On
Base Temperature (C): 220
Mode: Split
Split Flow: On
Split Flow Flow (ml/min): 50
Splitless Time (min): 1.00
Surge Pressure: Off
Surge Pressure (kPa): 3.00
Surge Duration (min): 0.00
Constant Purge: On
Stop Purge At: (min): 0.00

### Right Carrier Method

Mode: Constant Flow
Initial Value: On
Initial Value (ml/min): 1.20
Initial Time: 1.00
Gas Saver: On
Gas Saver Flow (ml/min): 20
Gas Saver Time: 5.00
Vacuum Compensation On

### Exemple 1.

Dans un autoclave agité, on a introduit à température ambiante, 65,60 g (712 mmol) de glycérol, 1.58 g (17,9 mmol) d'acide butyrique, 0,16 g (10 wt%) d'une résine Amberlyst 35 et 0,39 g (1.0 mol%) de Pd/C à 5%. L'autoclave a été ensuite pressurisé sous 50 bar de gaz dihydrogène H₂, puis a été chauffé à 120°C pendant 16h sous vive agitation. Après 16h de réaction, le milieu réactionnel a été ramené à la température ambiante. Le catalyseur a été filtré sur filtre millipore (0.45 µm) et a été rincé avec de l'éthanol absolu. Les solvants ont été évaporés et les produits organiques ont été extraits de la phase glycérol par extraction au CH₂Cl₂ (3 x 50 ml). La phase organique a été lavée à l'eau (2 x 30 ml) et le CH₂Cl₂ a été ensuite évaporé sous pression réduite. Le brut réactionnel a été purifié sur colonne de silice flash (éluant : cyclohexane/ acétate d'éthyle = 4 : 1 ∼ 1 : 1). Le 1-*O*-butyl-éther de glycérol a été obtenu avec un rendement isolé de 68%.

### Exemple 2.

Dans un autoclave agité, on a introduit à température ambiante, 68,37 g (742 mmol) de glycérol, 1.80 g (17,6 mmol) d'acide valérique, 0.18 g (10 wt%) d'une résine Amberlyst 35 et 0.39 g (1,0 mol%) de Pd/C à 5%. L'autoclave a été ensuite pressurisé sous 50 bar de gaz dihydrogène H₂, puis a été chauffé à 120°C pendant 16h sous vive agitation. Après 16h de réaction, le milieu réactionnel a été ramené à la température ambiante. Le catalyseur a été filtré sur filtre millipore (0,45 µm) et a été rincé avec de l'éthanol absolu. Les solvants ont été évaporés et les produits organiques ont été extraits de la phase glycérol par extraction au CH₂Cl₂ (3 x 50 ml). La phase organique a été lavée à l'eau (2 x 30 ml) et le CH₂Cl₂ a été ensuite évaporé sous pression réduite. Le brut réactionnel a été purifié sur colonne de silice flash (éluant : cyclohexane/ acétate d'éthyle = 4 : 1 ∼ 1 : 1). Le 1-*O*-pentyl-éther de glycérol a été obtenu avec un rendement isolé de 71%.

### Exemple 3.

Dans un autoclave agité, on a introduit à température ambiante, 65.73 g (714 mmol) de glycérol, 1.80 g (17.6 mmol) d'acide valérique, 0.18 g (10 wt%) d'une résine Amberlyst 35 et 0.39 g (1.0 mol%) de Pd/C à 5%. L'autoclave a été ensuite pressurisé sous 50 bar de gaz dihydrogène H₂, puis a été chauffé à 120°C pendant 16h sous vive agitation. Après 16h de réaction, le milieu réactionnel a été ramené à la température ambiante. Le catalyseur a été filtré sur filtre millipore (0.45 µm) et a été rincé avec de l'éthanol absolu. Les solvants ont été évaporés et les produits organiques ont été extraits de la phase glycérol par extraction au CH₂Cl₂ (3 x 50 ml). La phase organique a été lavée à l'eau (2 x 30 ml) et le CH₂Cl₂ a été ensuite évaporé sous pression réduite. Le brut réactionnel a été purifié sur colonne de silice flash (éluant : cyclohexane/ acétate d'éthyle = 4 : 1 ∼ 1 : 1). Le 1-*O*-pentyl-éther de glycérol a été obtenu avec un rendement isolé de 72%.

### Exemple 4.

Dans un autoclave agité, on a introduit à température ambiante, 65.76 g (714 mmol) de glycérol, 2.15 g (17.6 mmol) de valérate de méthyle, 0.23 g (10 wt%) d'acide camphosulfonique et 0.40 g (1.0 mol%) de Pd/C à 5%. L'autoclave a été ensuite pressurisé sous 50 bar de gaz dihydrogène H₂, puis a été chauffé à 140°C pendant 16h sous vive agitation. Après 16h de réaction, le milieu réactionnel a été ramené à la température ambiante. Le catalyseur a été filtré sur filtre millipore (0.45 µm) et a été rincé avec de l'éthanol absolu. Les solvants ont été évaporés et les produits organiques ont été extraits de la phase glycérol par extraction au CH₂Cl₂ (3 x 50 ml). La phase organique a été lavée à l'eau (2 x 30 ml) et le CH₂Cl₂ a été ensuite évaporé sous pression réduite. Le brut réactionnel a été purifié sur colonne de silice flash (éluant : cyclohexane/ acétate d'éthyle = 4 : 1 ∼ 1 : 1). Le 1-*O*-pentyl-éther de glycérol a été obtenu avec un rendement isolé de 58%.

### Exemple 5

Dans un autoclave agité, on a introduit à température ambiante, 55.2 g (600 mmol) de glycérol, 3.9 g (30 mmol) de caproate de méthyle, 0,39 g (10 wt%) d'acide camphosulfonique et 0,3 g (0.5 mol%) de Pd/C à 5%. L'autoclave a été ensuite pressurisé sous 10 bar de gaz dihydrogène H₂, puis a été chauffé à 140°C pendant 24h sous vive agitation. Après 24h de réaction, le milieu réactionnel a été ramené à la température ambiante. Le catalyseur a été filtré sur filtre millipore (0.45 µm) et a été rincé avec de l'éthanol absolu. Les solvants ont été évaporés et les produits organiques ont été extraits de la phase glycérol par extraction au CH₂Cl₂ (3 x 50 ml). La phase organique a été lavée à l'eau (2 x 30 ml) et le CH₂Cl₂ a été ensuite évaporé sous pression réduite. Le brut réactionnel a été analysé par GC/MS. Le 1-*O*-hexyl-éther de glycérol a été obtenu avec 22% de rendement GC.

### Exemple 6

Dans un autoclave agité, on a introduit à température ambiante, 55.2 g (600 mmol) de glycérol, 3,9 g (30 mmol) de caproate de méthyle, 0.4 g (10 wt%) d'acide camphosulfonique et 0.3 g (0.5 mol%) de Pd/C à 5%. L'autoclave a été ensuite pressurisé sous 20 bar de gaz dihydrogène H₂, puis a été chauffé à 140°C pendant 24h sous vive agitation. Après 24h de réaction, le milieu réactionnel a été ramené à la température ambiante. Le catalyseur a été filtré sur filtre millipore (0.45 µm) et a été rincé avec de l'éthanol absolu. Les solvants ont été ensuite évaporés et les produits organiques ont été extraits de la phase glycérol par extraction au CH₂Cl₂ (3 x 50 ml). La phase organique a été lavée à l'eau (2 x 30 ml) et le CH₂Cl₂ a été ensuite évaporé sous pression réduite. Le brut réactionnel a été analysé par GC/MS. Le 1-*O*-hexyl-éther de glycérol a été obtenu avec 30% de rendement GC.

### Exemple 7

Dans un autoclave agité, on a introduit à température ambiante, 55.2 g (600 mmol) de glycérol, 3,9 g (30 mmol) de caproate de méthyle, 0.4 g (10 wt%) d'acide camphosulfonique et 0.3 g (0.5 mol%) de Pd/C à 5%. L'autoclave a été ensuite pressurisé sous 30 bar de gaz dihydrogène H₂, puis a été chauffé à 140°C pendant 24h sous vive agitation. Après 24h de réaction, le milieu réactionnel a été ramené à la température ambiante. Le catalyseur a été ensuite filtré sur filtre millipore (0.45 µm) et a été rincé avec de l'éthanol absolu. Les solvants ont été ensuite évaporés et les produits organiques ont été extraits de la phase glycérol par extraction au CH₂Cl₂ (3 x 50 ml). La phase organique a été lavée à l'eau (2 x 20 ml) et le CH₂Cl₂ a été ensuite évaporé sous pression réduite. Le brut réactionnel a été analysé par GC/MS. Le 1-*O*-hexyl-éther de glycérol a été obtenu avec 50% de rendement GC.

### Exemple 8

Dans un autoclave agité, on a introduit à température ambiante, 55.2 g (600 mmol) de glycérol, 3,9 g (30 mmol) de caproate de méthyle, 0.4 g (10 wt%) d'acide camphosulfonique et 0.3 g (0.5 mol%) de Pd/C à 5%. L'autoclave a été ensuite pressurisé sous 40 bar de gaz dihydrogène H₂, puis a été chauffé à 140°C pendant 24h sous vive agitation. Après 24h de réaction, le milieu réactionnel a été ramené à la température ambiante. Le catalyseur a été ensuite filtré sur filtre millipore (0.45 µm) et a été rincé avec de l'éthanol absolu. Les solvants ont été ensuite évaporés et les produits organiques ont été extraits de la phase glycérol par extraction au CH₂Cl₂ (3 x 50 ml). La phase organique a été lavée à l'eau (2 x 20 ml) et le CH₂Cl₂ a été ensuite évaporé sous pression réduite. Le brut réactionnel a été analysé par GC/MS. Le 1-*O*-hexyl-éther de glycérol a été obtenu avec 55% de rendement GC.

### Exemple 9

Dans un autoclave agité, on a introduit à température ambiante, 55.2 g (600 mmol) de glycérol, 3,9 g (30 mmol) de caproate de méthyle, 0.4 g (10 wt%) d'acide camphosulfonique et 0.6 g (1 mol%) de Pd/C à 5%. L'autoclave a été ensuite pressurisé sous 50 bar de gaz dihydrogène H₂, puis a été chauffé à 140°C pendant 24h sous vive agitation. Après 24h de réaction, le milieu réactionnel a été ramené à la température ambiante. Le catalyseur a été ensuite filtré sur filtre millipore (0.45 µm) et a été rincé avec de l'éthanol absolu. Les solvants ont été ensuite évaporés et les produits organiques ont été extraits de la phase glycérol par extraction au CH₂Cl₂ (3 x 50 ml). La phase organique a été lavée à l'eau (2 x 20 ml) et le CH₂Cl₂ a été ensuite évaporé sous pression réduite. Le brut réactionnel a été analysé par GC/MS. Le 1-*O*-hexyl-éther de glycérol a été obtenu avec 58% de rendement GC.

### Exemple 10

Dans un autoclave agité, on a introduit à température ambiante, 66.75 g (725 mmol) de glycérol, 2.05 g (17.6 mmol) d'acide caproïque, 0.20 g (10 wt%) d'une résine Amberlyst 35 et 0.39 g (1.0 mol%) de Pd/C à 5%. L'autoclave a été ensuite pressurisé sous 50 bar de gaz dihydrogène H₂, puis a été chauffé à 120°C pendant 16h sous vive agitation. Après 16h de réaction, le milieu réactionnel a été ramené à la température ambiante. Le catalyseur a été ensuite filtré sur filtre millipore (0.45 µm) et a été rincé avec de l'éthanol absolu. Les solvants ont été ensuite évaporés et les produits organiques ont été extraits de la phase glycérol par extraction au CH₂Cl₂ (3 x 50 ml). La phase organique a été lavée à l'eau (2 x 20 ml) et le CH₂Cl₂ a été ensuite évaporé sous pression réduite. Le brut réactionnel a été purifié sur colonne de silice flash (éluant : cyclohexane/ acétate d'éthylé = 4 : 1 ∼ 1 : 1). Le 1-*O*-hexyl-éther de glycérol a été obtenu avec un rendement isolé de 70%.

### Exemple 11

Dans un autoclave agité, on a introduit à température ambiante, 65.50 g (711 mmol) de glycérol, 2.29 g (17.6 mmol) de caproate de méthyle, 0.23 g (10 wt%) d'acide camphosulfonique et 0.39 g (1 mol%) de Pd/C à 5%. L'autoclave a été ensuite pressurisé sous 50 bar de gaz dihydrogène H₂, puis a été chauffé à 140°C pendant 16h sous vive agitation. Après 16h de réaction, le milieu réactionnel a été ramené à la température ambiante. Le catalyseur a été ensuite filtré sur filtre millipore (0.45 µm) et a été rincé avec de l'éthanol absolu. Les solvants ont été ensuite évaporés et les produits organiques ont été extraits de la phase glycérol par extraction au CH₂Cl₂ (3 x 50 ml). La phase organique a été lavée à l'eau (2 x 20 ml) et le CH₂Cl₂ a été ensuite évaporé sous pression réduite. Le brut réactionnel a été analysé par GC/MS. Le 1-*O*-hexyl-éther de glycérol a été obtenu avec 83% de rendement GC.

### Exemple 12

Dans un autoclave agité, on a introduit à température ambiante, 65.84 g (715 mmol) de glycérol, 2.29 g (17.6 mmol) de caproate de méthyle, 0.23 g (10 wt%) d'acide camphosulfonique et 0.39 g (1 mol%) de Pd/C à 5%. L'autoclave a été ensuite pressurisé sous 50 bar de gaz dihydrogène H₂, puis a été chauffé à 120°C pendant 16h sous vive agitation. Après 16h de réaction, le milieu réactionnel a été ramené à la température ambiante. Le catalyseur a été ensuite filtré sur filtre millipore (0.45 µm) et a été rincé avec de l'éthanol absolu. Les solvants ont été ensuite évaporés et les produits organiques ont été extraits de la phase glycérol par extraction au CH₂Cl₂ (3 x 50 ml). La phase organique a été lavée à l'eau (2 x 20 ml) et le CH₂Cl₂ a été ensuite évaporé sous pression réduite. Le brut réactionnel a été analysé par GC/MS. Le 1-*O*-hexyl-éther de glycérol a été obtenu avec 59% de rendement GC.

### Exemple 13

Dans un autoclave agité, on a introduit à température ambiante, 65.61 g (712 mmol) de glycérol, 2.29 g (17.6 mmol) de caproate de méthyle, 0.23 g (10 wt%) d'une résine Amberlyst 15 et 0.23 g (1 mol%) de Pd/C à 5%. L'autoclave a été ensuite pressurisé sous 50 bar de gaz dihydrogène H₂, puis a été chauffé à 140°C pendant 16h sous vive agitation. Après 16h de réaction, le milieu réactionnel a été ramené à la température ambiante. Le catalyseur a été ensuite filtré sur filtre millipore (0.45 µm) et a été rincé avec de l'éthanol absolu. Les solvants ont été ensuite évaporés et les produits organiques ont été extraits de la phase glycérol par extraction au CH₂Cl₂ (3 x 50 ml). La phase organique a été lavée à l'eau (2 x 20 ml) et le CH₂Cl₂ a été ensuite évaporé sous pression réduite. Le brut réactionnel a été analysé par GC/MS. Le 1-*O*-hexyl-éther de glycérol a été obtenu avec 62% de rendement GC.

### Exemple 14

Dans un autoclave agité, on a introduit à température ambiante, 66.47 g (722 mmol) de glycérol, 2.53 g (17.5 mmol) d'acide octanoïque, 0.25 g (10 wt%) d'acide camphosulfonique et 0.39 g (1.0 mol%) de Pd/C à 5%. L'autoclave a été ensuite pressurisé sous 50 bar de gaz dihydrogène H₂, puis a été chauffé à 140°C pendant 16h sous vive agitation. Après 16h de réaction, le milieu réactionnel a été ramené à la température ambiante. Le catalyseur a été filtré sur filtre millipore (0.1 µm) et a été rincé avec de l'éthanol absolu. Les solvants ont été évaporés et les produits organiques ont été extraits de la phase glycérol par extraction au CH₂Cl₂ (3 x 50 ml). La phase organique a été lavée à l'eau (2 x 30 ml) et le CH₂Cl₂ a été ensuite évaporé sous pression réduite. Le brut réactionnel a été purifié sur colonne de silice flash (éluant : cyclohexane/ acétate d'éthyle = 4 : 1 ∼ 1 : 1). Le 1-*O*-octanyl-éther de glycérol a été obtenu avec un rendement isolé de 52%.

### Exemple 15

Dans un autoclave agité, on a introduit à température ambiante, 65.63 g (713 mmol) de glycérol, 2.54 g (17.6 mmol) d'acide octanoïque, 0.26 g (10 wt%) d'une résine Amberlyst 35 et 0.39 g (1.0 mol%) de Pd/C à 5%. L'autoclave a été ensuite pressurisé sous 50 bar de gaz dihydrogène H₂, puis a été chauffé à 120°C pendant 16h sous vive agitation. Après 16h de réaction, le milieu réactionnel a été ramené à la température ambiante. Le catalyseur a été filtré sur filtre millipore (0.1 µm) et a été rincé avec de l'éthanol absolu. Les solvants ont été évaporés et les produits organiques ont été extraits de la phase glycérol par extraction au CH₂Cl₂ (3 x 50 ml). La phase organique a été lavée à l'eau (2 x 30 ml) et le CH₂Cl₂ a été ensuite évaporé sous pression réduite. Le brut réactionnel a été purifié sur colonne de silice flash (éluant : cyclohexane/ acétate d'éthyle = 4 : 1 ∼ 1 : 1). Le 1-*O-*octanyl-éther de glycérol a été obtenu avec un rendement isolé de 48%.

### Exemple 16

Dans un autoclave agité, on a introduit à température ambiante, 69 g (750 mmol) de glycérol, 3.9 g (25 mmol) d'acide pelargonique, 0.39 g (10 wt%) d'acide camphosulfonique et 0.63 g (1 mol%) de Pd/C à 5%. L'autoclave a été ensuite pressurisé sous 40 bar de gaz dihydrogène H₂, puis a été chauffé à 140°C pendant 24h sous vive agitation. Après 24h de réaction, le milieu réactionnel a été ramené à la température ambiante. Le catalyseur a été ensuite filtré sur filtre millipore (0.45 µm) et a été rincé avec de l'éthanol absolu. Les solvants ont été ensuite évaporés et les produits organiques ont été extraits de la phase glycérol par extraction au CH₂Cl₂ (3 x 100 ml). La phase organique a été lavée à l'eau (2 x 30 ml) et le CH₂Cl₂ a été ensuite évaporé sous pression réduite. Le brut réactionnel a été analysé par GC/MS et le 1-*O*-nonyl éther de glycérol a été obtenu avec un rendement GC de 43%.

### Exemple 17

Dans un autoclave agité, on a introduit à température ambiante, 55.2 g (600 mmol) de glycérol, 51.6 g (30 mmol) de pelargonate de méthyle, 0.039 g (10 wt%) d'acide camphosulfonique et 0.64 g (1 mol%) de Pd/C à 5%. L'autoclave a été ensuite pressurisé sous 10 bar de gaz dihydrogène H₂, puis a été chauffé à 145°C pendant 24h sous vive agitation. Après 24h de réaction, le milieu réactionnel a été ramené à la température ambiante. Le catalyseur a été ensuite filtré sur filtre millipore (0.45 µm) et a été rincé avec de l'éthanol absolu. Les solvants ont été ensuite évaporés et les produits organiques ont été extraits de la phase glycérol par extraction au CH₂Cl₂ (3 x 50 ml). La phase organique a été lavée à l'eau (2 x 20 ml) et le CH₂Cl₂ a été ensuite évaporé sous pression réduite. Le 1-*O*-nonyl-éther de glycérol a été obtenu par purification sur colonne de silice flash avec un mélange Acétate d'éthyle/cyclohexane 1/4 à 1/1 comme éluant avec un rendement isolé de 20%.

### Exemple 18

Dans un autoclave agité, on a introduit à température ambiante, 65.35 g (710 mmol) de glycérol, 3.01 g (17.5 mmol) d'acide décanoïque, 0.30 g (10 wt%) d'une résine Amberlyst 35 et 0.39 g (1.0 mol%) de Pd/C à 5%. L'autoclave a été ensuite pressurisé sous 50 bar de gaz dihydrogène H₂, puis a été chauffé à 120°C pendant 16h sous vive agitation. Après 16h de réaction, le milieu réactionnel a été ramené à la température ambiante. Le catalyseur a été filtré sur filtre millipore (0.1 µm) et a été rincé avec de l'éthanol absolu. Les solvants ont été évaporés et les produits organiques ont été extraits de la phase glycérol par extraction au CH₂Cl₂ (3 x 50 ml). La phase organique a été lavée à l'eau (2 x 30 ml) et le CH₂Cl₂ a été ensuite évaporé sous pression réduite. Le brut réactionnel a été purifié sur colonne de silice flash (éluant : cyclohexane/ acétate d'éthyle = 4 : 1 ∼ 1 : 1). Le 1-*O*-décanyl-éther de glycérol a été obtenu avec un rendement isolé de 46%.

### Exemple 19

Dans un autoclave agité, on a introduit à température ambiante, 65.43 g (711 mmol) de glycérol, 3.50 g (17.5 mmol) d'acide dodécanoïque, 0.35 g (10 wt%) d'une résine Amberlyst 35 et 0.39 g (1.0 mol%) de Pd/C à 5%. L'autoclave a été ensuite pressurisé sous 50 bar de gaz dihydrogène H₂, puis a été chauffé à 120°C pendant 16h sous vive agitation. Après 16h de réaction, le milieu réactionnel a été ramené à la température ambiante. Le catalyseur a été filtré sur filtre millipore (0.1 µm) et a été rincé avec de l'éthanol absolu. Les solvants ont été évaporés et les produits organiques ont été extraits de la phase glycérol par extraction au CH₂Cl₂ (3 x 50 ml). La phase organique a été lavée à l'eau (2 x 30 ml) et le CH₂Cl₂ a été ensuite évaporé sous pression réduite. Le brut réactionnel a été purifié sur colonne de silice flash (éluant : cyclohexane/ acétate d'éthyle = 5 : 1 ∼ 1 : 1). Le 1-*O*-dodécanyl-éther de glycérol a été obtenu avec un rendement isolé de 42%.

### Exemple 20

Dans un autoclave agité, on a introduit à température ambiante, 67 g (728 mmol) de glycérol, 8.2 g (36 mmol) de tridécylate de méthyle, 0.82 g (10 wt%) d'acide camphosulfonique et 0.74 g (1.0 mol%) de Pd/C à 5%. L'autoclave a été ensuite pressurisé sous 50 bar de gaz dihydrogène H₂, puis a été chauffé à 140°C pendant 24h sous vive agitation. Après 24h de réaction, le milieu réactionnel a été ramené à la température ambiante. Le catalyseur a été ensuite filtré sur filtre millipore (0.45 µm) et a été rincé avec de l'éthanol absolu. Les solvants ont été ensuite évaporés et les produits organiques ont été extraits de la phase glycérol par extraction au CH₂Cl₂ (3 x 50 ml). La phase organique a été lavée à l'eau (2 x 20 ml) et le CH₂Cl₂ a été ensuite évaporé sous pression réduite. Le brut réactionnel a été analysé par GC/MS. Le 1-*O*-tridécyl-éther de glycérol a été obtenu avec 16% de rendement GC.

### Exemple 21

Dans un autoclave agité, on a introduit à température ambiante, 67 g (728 mmol) de glycérol, 8.2 g (36 mmol) d'acide myristique, 0.21 g (5 wt%) d'acide camphosulfonique et 0.74 g (1 mol%) de Pd/C à 5%. L'autoclave a été ensuite pressurisé sous 40 bar de gaz dihydrogène H₂, puis a été chauffé à 140°C pendant 24h sous vive agitation. Après 24h de réaction, le milieu réactionnel a été ramené à la température ambiante. Le catalyseur a été ensuite filtré sur filtre millipore (0.45 µm) et a été rincé avec de l'éthanol absolu. Les solvants ont été ensuite évaporés et les produits organiques ont été extraits de la phase glycérol par extraction au CH₂Cl₂ (3 x 100 ml). La phase organique a été lavée à l'eau (2 x 30 ml) et le CH₂Cl₂ a été ensuite évaporé sous pression réduite. Le brut réactionnel a été analysé par GC/MS et le 1-*O*-tétradécyl éther de glycérol a été obtenu avec un rendement GC de 71% et, après purification sur colonne de silice flash avec Acétate d'éthyle/cyclohexane 1/4 à 1/1 comme éluant, a été obtenu avec un rendement isolé de 36%.

### Exemple 22

Dans un autoclave agité, on a introduit à température ambiante, 65.41 g (710 mmol) de glycérol, 4.01 g (17.6 mmol) d'acide myristique, 0.40 g (10 wt%) d'une résine Amberlyst 35 et 0.39 g (1.0 mol%) de Pd/C à 5%. L'autoclave a été ensuite pressurisé sous 50 bar de gaz dihydrogène H₂, puis a été chauffé à 120°C pendant 16h sous vive agitation. Après 16h de réaction, le milieu réactionnel a été ramené à la température ambiante. Le catalyseur a été filtré sur filtre millipore (0.1 µm) et a été rincé avec de l'éthanol absolu. Les solvants ont été évaporés et les produits organiques ont été extraits de la phase glycérol par extraction au CH₂Cl₂ (3 x 50 ml). La phase organique a été lavée à l'eau (2 x 30 ml) et le CH₂Cl₂ a été ensuite évaporé sous pression réduite. Le brut réactionnel a été purifié sur colonne de silice flash (éluant : cyclohexane/ acétate d'éthyle = 5 : 1 ∼ 1 : 1). Le 1-*O*-tétradécanyl-éther de glycérol a été obtenu avec un rendement isolé de 41%.

### Exemple 23

Dans un autoclave agité, on a introduit à température ambiante, 69.14 g (751 mmol) de glycérol, 12.34 g (61.6 mmol) d'acide palmitique, 1.29 g (10 wt%) d'acide camphosulfonique et 1.39 g (1.0 mol%) de Pd/C à 5%. L'autoclave a été ensuite pressurisé sous 50 bar de gaz dihydrogène H₂, puis a été chauffé à 140°C pendant 16h sous vive agitation. Après 16h de réaction, le milieu réactionnel a été ramené à la température ambiante. Le catalyseur a été filtré sur filtre millipore (0.1 µm) et a été rincé avec de l'éthanol absolu. Les solvants ont été évaporés et les produits organiques ont été extraits de la phase glycérol par extraction au CH₂Cl₂ (3 x 50 ml). La phase organique a été lavée à l'eau (2 x 30 ml) et le CH₂Cl₂ a été ensuite évaporé sous pression réduite. Le brut réactionnel a été purifié sur colonne de silice flash (éluant : cyclohexane/ acétate d'éthyle = 6 : 1 ∼ 1 : 1). Le 1-*O*-hexadécanyl-éther de glycérol a été obtenu avec un rendement isolé de 28%.

### Exemple 24

Dans un autoclave agité, on a introduit à température ambiante, 68.13 g (740 mmol) de glycérol, 4.40 g (17.2 mmol) d'acide palmitique, 0.45 g (10 wt%) d'une résine Amberlyst 35 et 0.39 g (1.0 mol%) de Pd/C à 5%. L'autoclave a été ensuite pressurisé sous 50 bar de gaz dihydrogène H₂, puis a été chauffé à 120°C pendant 16h sous vive agitation. Après 16h de réaction, le milieu réactionnel a été ramené à la température ambiante. Le catalyseur a été filtré sur filtre millipore (0.1 µm) et a été rincé avec de l'éthanol absolu. Les solvants ont été évaporés et les produits organiques ont été extraits de la phase glycérol par extraction au CH₂Cl₂ (3 x 50 ml). La phase organique a été lavée à l'eau (2 x 30 ml) et le CH₂Cl₂ a été ensuite évaporé sous pression réduite. Le brut réactionnel a été purifié sur colonne de silice flash (éluant : cyclohexane/ acétate d'éthyle = 6 : 1 ∼ 1 : 1). Le 1-*O*-hexadécanyl-éther de glycérol a été obtenu avec un rendement isolé de 22%.

### Exemple 25

Dans un autoclave agité, on a introduit à température ambiante, 92 g (1 mol) de glycérol, 7.1 g (25 mmol) d'acide Stéarique, 0.71 g (10 wt%) d'acide camphosulfonique et 0.53 g (1 mol%) de Pd/C à 5%. L'autoclave a été ensuite pressurisé sous 50 bar de gaz dihydrogène H₂, puis a été chauffé à 140°C pendant 24h sous vive agitation. Après 24h de réaction, le milieu réactionnel a été ramené à la température ambiante. Le catalyseur a été ensuite filtré sur filtre millipore (0.45 µm) et a été rincé avec de l'éthanol absolu. Les solvants ont été ensuite évaporés et les produits organiques ont été extraits de la phase glycérol par extraction au CH₂Cl₂ (3 x 100 ml). La phase organique a été lavée à l'eau (2 x 30 ml) et le CH₂Cl₂ a été ensuite évaporé sous pression réduite. Le 1-*O-*octadécyl éther de glycérol a été obtenu par purification sur colonne de silice flash avec Acétate d'éthyle/cyclohexane 1/4 à 1/1 comme éluant, avec un rendement isolé de 30%.

### Exemple 26

Dans un autoclave agité, on a introduit à température ambiante, 122.9 g (1.33 mol) de glycérol, 12.5 g (66.5 mmol) d'acide azélaïque, 1.25 g (10 wt%) d'acide camphosulfonique et 3.0 g (2 mol%) de Pd/C à 5%. L'autoclave a été ensuite pressurisé sous 60 bar de gaz dihydrogène H₂, puis a été chauffé à 140°C pendant 24h sous vive agitation. Après 24h de réaction, le milieu réactionnel a été ramené à la température ambiante. Le catalyseur a été ensuite filtré sur filtre millipore (0.45 µm) et a été rincé avec de l'éthanol absolu. Les solvants ont été ensuite évaporés et les produits organiques ont été extraits de la phase glycérol par extraction au CH₂Cl₂ (4 x 200 ml). Le CH₂Cl₂ a été ensuite évaporé sous pression réduite. Le brut réactionnel a été analysé par GC/MS. Le 1,9-*O,O*-nonyl-diéther de glycérol a été obtenu avec 30% de rendement GC.

### Exemple 27

Dans un autoclave agité, on a introduit à température ambiante, 101.7 g (1.11 mol) de glycérol, 6.55 g (21 mmol) de diacide D18 :1, 0.65 g (10 wt%) d'acide camphosulfonique et 0.8 g (1.8 mol%) de Pd/C à 5%. L'autoclave a été ensuite pressurisé sous 50 bar de gaz dihydrogène H₂, puis a été chauffé à 140°C pendant 26h sous vive agitation. Après 26h de réaction, le milieu réactionnel a été ramené à la température ambiante. Le catalyseur a été ensuite filtré sur filtre millipore (0.45 µm) et a été rincé avec un mélange de CH₂Cl₂ et d'éthanol absolu. Les solvants ont été ensuite évaporés et les produits organiques ont été extraits de la phase glycérol par extraction au CH₂Cl₂ (3 x 300 ml). Le CH₂Cl₂ a été ensuite évaporé sous pression réduite. Le brut réactionnel a été analysé par GC/MS. Le 1,18-*O,O*-octadécyl-diéther de glycérol a été obtenu avec 10% de rendement GC.

### Exemple 28

Dans un autoclave agité, on a introduit à température ambiante, 65.66 g (395 mmol) de diglycérol, 1.34 g (11.5 mmol) d'acide caproïque, 0.13 g (10 wt%) d'une résine Amberlyst 35 et 0.26 g (1.0 mol%) de Pd/C à 5%. L'autoclave a été ensuite pressurisé sous 50 bar de gaz dihydrogène H₂, puis a été chauffé à 120°C pendant 16h sous vive agitation. Après 16h de réaction, le milieu réactionnel a été ramené à la température ambiante. Le catalyseur a été filtré sur filtre millipore (0.1 µm) et a été rincé avec de l'éthanol absolu. Les solvants ont été évaporés et les produits organiques ont été extraits de la phase glycérol par extraction au CH₂Cl₂ (3 x 50 ml). La phase organique a été lavée à l'eau (2 x 30 ml) et le CH₂Cl₂ a été ensuite évaporé sous pression réduite. Le brut réactionnel a été purifié sur colonne de silice flash (éluant : cyclohexane/ acétate d'éthyle = 2 : 1 ∼ acétate d'éthyle 100 %). Le 1-*O*-hexyl-éther de diglycérol a été obtenu avec un rendement isolé de 57%.

### Exemple 29

Dans un autoclave agité, on a introduit à température ambiante, 65.35 g (393 mmol) de diglycérol, 2.26 g (13.1 mmol) d'acide décanoïque, 0.23 g (10 wt%) d'une résine Amberlyst 35 et 0.28 g (1.0 mol%) de Pd/C à 5%. L'autoclave a été ensuite pressurisé sous 50 bar de gaz dihydrogène H₂, puis a été chauffé à 120°C pendant 16h sous vive agitation. Après 16h de réaction, le milieu réactionnel a été ramené à la température ambiante. Le catalyseur a été filtré sur filtre millipore (0.1 µm) et a été rincé avec de l'éthanol absolu. Les solvants ont été évaporés et les produits organiques ont été extraits de la phase glycérol par extraction au CH₂Cl₂ (3 x 50 ml). La phase organique a été lavée à l'eau (2 x 30 ml) et le CH₂Cl₂ a été ensuite évaporé sous pression réduite. Le brut réactionnel a été purifié sur colonne de silice flash (éluant : cyclohexane/ acétate d'éthyle = 2 : 1 ∼ 1 : 1). Le 1-*O*-décanyl-éther de diglycérol a été obtenu avec un rendement isolé de 37%.

### Exemples 30, 31 et 32 : Recyclage du système catalytique

### Exemple 30

Dans un autoclave agité, on a introduit à température ambiante, 65.85 g (715 mmol) de glycérol, 1.80 g (17.6 mmol) d'acide valérique, 0.18 g (10 wt%) d'une résine Amberlyst 35 et 0.39 g (1.0 mol%) de Pd/C à 5%. L'autoclave a été ensuite pressurisé sous 50 bar de gaz dihydrogène H₂, puis a été chauffé à 120°C pendant 16h sous vive agitation. Après 16h de réaction, le milieu réactionnel a été ramené à la température ambiante. Le catalyseur a été filtré sur filtre millipore (0.1 µm) et a été rincé avec de l'éthanol absolu. Les solvants ont été évaporés et les produits organiques ont été extraits de la phase glycérol par extraction au CH₂Cl₂ (3 x 50 ml). La phase organique a été lavée à l'eau (2 x 30 ml) et le CH₂Cl₂ a été ensuite évaporé sous pression réduite. Le brut réactionnel a été purifié sur colonne de silice flash (éluant : cyclohexane/ acétate d'éthyle = 4 : 1 ∼ 1 : 1). Le 1-*O*-pentyl-éther de glycérol a été obtenu avec un rendement isolé de 69%.

Après la réaction, la mixture de catalyseurs (résine Amberlyst 35 et Pd/C) a été immergée dans 5ml de méthanol, puis transférée dans l'autoclave. Celle-ci a été séchée sous un flux d'argon pendant 4h à température ambiante. Après évaporation totale du solvant, les substrats (glycérol et acide valérique) ont été ajoutés pour une nouvelle réaction (cf. exemple 31).

### Exemple 31

Dans un autoclave agité, on a introduit à température ambiante sur la mixture de catalyseurs recyclés de l'exemple 30 (Amberlyst 35 et Pd/C), 66.86 g (726 mmol) de glycérol et 1.80 g (17.6 mmol) d'acide valérique. L'autoclave a été ensuite pressurisé sous 50 bar de gaz dihydrogène H₂, puis a été chauffé à 120°C pendant 16h sous vive agitation. Après 16h de réaction, le milieu réactionnel a été ramené à la température ambiante. Le catalyseur a été filtré sur filtre millipore (0.1 µm) et a été rincé avec de l'éthanol absolu. Les solvants ont été évaporés et les produits organiques ont été extraits de la phase glycérol par extraction au CH₂Cl₂ (3 x 50 ml). La phase organique a été lavée à l'eau (2 x 30 ml) et le CH₂Cl₂ a été ensuite évaporé sous pression réduite. Le brut réactionnel a été purifié sur colonne de silice flash (éluant : cyclohexane/ acétate d'éthyle = 4 : 1 ∼ 1 : 1). Le 1-*O-*pentyl-éther de glycérol a été obtenu avec un rendement isolé de 63%.

Après la réaction, la mixture de catalyseurs (résine Amberlyst 35 et Pd/C) a été immergée dans 5ml de méthanol, puis transférée dans l'autoclave. Celle-ci a été séchée sous un flux d'argon pendant 4h à température ambiante. Après évaporation totale des solvants, les substrats (glycérol et acide valérique) ont été ajoutés pour une nouvelle réaction (cf. exemple 32).

### Exemple 32

Dans un autoclave agité, on a introduit à température ambiante sur la mixture de catalyseurs recyclés de l'exemple 31 (Amberlyst 35 et Pd/C), 65.56 g (712 mmol) de glycérol et 1.80 g (17.6 mmol) d'acide valérique. L'autoclave a été ensuite pressurisé sous 50 bar de gaz dihydrogène H₂, puis a été chauffé à 120°C pendant 16h sous vive agitation. Après 16h de réaction, le milieu réactionnel a été ramené à la température ambiante. Le catalyseur a été filtré sur filtre millipore (0.1 µm) et a été rincé avec de l'éthanol absolu. Les solvants ont été évaporés et les produits organiques ont été extraits de la phase glycérol par extraction au CH₂Cl₂ (3 x 50 ml). La phase organique a été lavée à l'eau (2 x 30 ml) et le CH₂Cl₂ a été ensuite évaporé sous pression réduite. Le brut réactionnel a été purifié sur colonne de silice flash (éluant : cyclohexane/ acétate d'éthyle = 4 : 1 ∼ 1 : 1). Le 1-*O*-pentyl-éther de glycérol a été obtenu avec un rendement isolé de 72%.

Les catalyseurs ont été recyclés trois fois sans perte d'activité, ni de rendement, ni de sélectivité.

## Revendications

1. Procédé de préparation en une seule étape d'un éther de polyol de formule I **caractérisé en ce qu'**il comprend une étape d'alkylation réductrice faisant intervenir un composé de formule générale II et un composé de formule générale III : 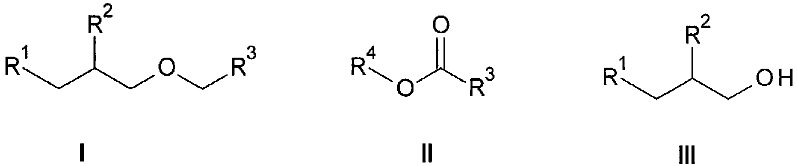 dans lesquelles :
- R¹ et R² représentent indépendamment l'un de l'autre des groupes choisis parmi un atome d'hydrogène, un groupement - OH ou -(OCH₂-CH(OH)-CH₂)ₚ-OH avec 1≤p≤10;
- R⁴ représente un atome d'hydrogène ou un groupe choisi parmi un groupe alkyle en C₁-C₅₀, un groupe alcènyle en C₂-C₅₀, un aryle, un cycloalkyle en C₃-C₈, un groupe -CH₂-CH[O-[C(=O)]ₙ-R₅]-CH₂-O-[C(=O)]ₘ-R₆, ledit groupe pouvant être non-substitué ou substitué;
- R³, R⁵ et R⁶ représentent indépendamment les uns des autres des groupes choisis parmi un alkyle en C₁-C₅₀, un alcènyle en C₂-C₅₀, un aryle, un cycloalkyle en C₃-C₈, lesdits groupes pouvant être non-substitués ou substitués;
- n et m sont indépendants l'un de l'autre et sont égaux à 0 ou 1 ;
- R⁵ est un atome d'hydrogène lorsque n=0;
- R⁶ est un atome d'hydrogène lorsque m=0 ;
et dans lesquelles au moins un de R¹ et R² est un groupe hydroxyle -OH ladite étape d'alkylation réductrice étant réalisée :
- en présence d'un catalyseur métallique choisi parmi le palladium, le rhodium, le ruthénium, le cobalt, le platine, l'iridium ou le nickel, ledit catalyseur étant éventuellement associé à un support choisi parmi le charbon, l'alumine, la silice ou l'aluminosilicate ;
- en présence d'un catalyseur acide ;et
- en présence d'un gaz réducteur, de préférence le dihydrogène.

2. Procédé selon la revendication 1 **caractérisé en ce que** ledit catalyseur métallique précité est utilisé en quantité molaire inférieure à 1 % par rapport à la quantité molaire de composé de formule générale II.

3. Procédé selon l'une quelconque des revendications 1 ou 2 **caractérisé en ce que** ladite étape d'alkylation réductrice est réalisée à une température inférieure à 200°C, de préférence inférieure à 180°C et encore plus préférentiellement inférieure à 150°C.

4. Procédé selon l'une quelconque des revendications 1 à 3 **caractérisé en ce que** ledit catalyseur acide est choisi parmi les acides de Lewis ou de Bronsted.

5. Procédé selon la revendication 4 **caractérisé en ce que** ledit catalyseur acide est choisi parmi l'acide camphosulphonique, l'acide paratoluene sulfonique, les résines sulfoniques acides, l'acide trifluoroacétique, la silice, la silice alumine, l'acide phosphorique supporté sur silice, BF₃.Et₂O ou les zéolithes.

6. Procédé selon l'une quelconque des revendications 1 à 5 **caractérisé en ce que** ledit catalyseur acide est présent en quantité inférieure à 10% en masse, de préférence inférieure à 5%, par rapport à la masse totale de composé de formule générale II.

7. Procédé selon l'une quelconque des revendications 1 à 6 **caractérisé en ce que** ledit catalyseur acide est un catalyseur acide se présentant sur support solide, formant, avec ledit catalyseur métallique, un système catalytique recyclable.

8. Procédé selon l'une quelconque des revendications 1 à 7 **caractérisé en ce que** ledit catalyseur acide se présentant sur support solide est une résine sulfonique acide.

9. Procédé selon l'une quelconque des revendications 1 à 8 **caractérisé en ce que** la réaction est réalisée sous une pression de dihydrogène comprise entre 1 et 150 bars, de préférence entre 1 et 100 bars, de préférence entre 10 et 50 bars, de préférence encore entre 30 et 50 bars.

10. Procédé selon l'une quelconque des revendications 1 à 9 **caractérisé en ce que** dans les formules I, II et III précitées :
- R¹ et R² représentent indépendamment l'un de l'autre des groupes choisis parmi un atome d'hydrogène, un groupement - OH, -(OCH₂-CH(OH)-CH₂)ₚ-OH avec 1≤p≤10;
- R³ représente un groupe choisi parmi un alkyle en C₁-C₅₀ et un alcènyle en C₂-C₅₀ ;
- R⁴ représente un groupe choisi parmi un atome d'hydrogène et un groupe alkyle en C₁-C₅ ;
et dans lesquelles au moins un de R¹ et R² est un groupe hydroxyle -OH.

11. Procédé selon l'une quelconque des revendications 1 à 10 **caractérisé en ce que** dans les formules I et II précitées:
- R³ représente un groupe choisi parmi un alkyle en C₁-C₂₈, de préférence en C₁-C₂₄, un alcènyle en C₂-C₂₈, de préférence en C₂-C₂₄;

12. Procédé selon l'une quelconque des revendications 1 à 11 **caractérisé en ce que** dans les formules I et III précitées R¹ et R² représentent tous les deux un groupement -OH.

13. Procédé selon l'une quelconque des revendications 1 à 12 **caractérisé en ce que** ledit composé de formule générale II est choisi parmi l'acide valérique, l'acide caproïque, l'acide adipique, l'acide pélargonique, l'acide azélaïque, l'acide brassylique, l'acide myristique, l'acide stéarique, l'acide oléique, l'acide 9Z-octadécènedioïque, l'acide érucique et les esters de méthyle correspondants.

## Patentansprüche

1. Verfahren zur Herstellung eines Polyolethers der Formel I in einem einzigen Schritt, **dadurch gekennzeichnet, dass** es einen Schritt der reduktiven Alkylierung aufweist, bei dem eine Verbindung der allgemeinen Formel II und eine Verbindung der allgemeinen Formel III verwendet werden: 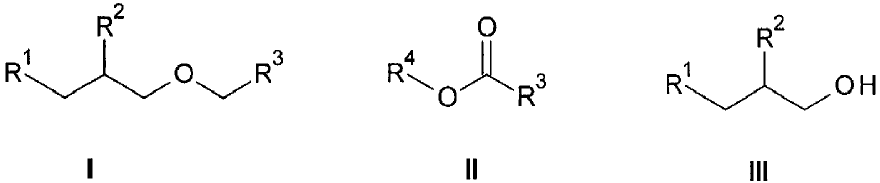 worin:
- R¹ und R² unabhängig voneinander Gruppen ausgewählt aus einem Wasserstoffatom, einer OH-Gruppe oder einer (OCH₂-CH(OH)-CH₂)ₚ-OH-Gruppe mit 1 ≤ p ≤ 10 darstellen,
- R⁴ ein Wasserstoffatom oder eine Gruppe ausgewählt aus einer C₁-C₅₀-Alkylgruppe, einer C₂-C₅₀-Alkenylgruppe, einem Aryl, einem C₃-C₈-Cycloalkyl, einer CH₂-CH[O-[C(=O)]ₙ- R₅]-CH₂-O-[C(=O)]ₘ-R₆-Gruppe darstellt, wobei die Gruppe unsubstituiert oder substituiert sein kann,
- R³, R ⁵ und R⁶ unabhängig voneinander Gruppen ausgewählt aus einem C₁-C₅₀-Alkyl, einem C₂-C₅₀-Alkenyl, einem Aryl, einem C₃-C₈-Cycloalkyl darstellen, wobei die Gruppen unsubstituiert oder substituiert sein können,
- n und m unabhängig voneinander gleich 0 oder 1 sind,
- R⁵ ein Wasserstoffatom ist, wenn n = 0,
- R⁶ ein Wasserstoffatom ist, wenn m = 0,
und wobei mindestens eines von R¹ und R² eine Hydroxylgruppe -OH ist, wobei der Schritt der reduktiven Alkylierung durchgeführt wird:
- in Gegenwart eines Metallkatalysators, ausgewählt aus Palladium, Rhodium, Ruthenium, Kobalt, Platin, Iridium oder Nickel, wobei der Katalysator gegebenenfalls mit einem Träger, ausgewählt aus Kohle, Aluminiumoxid, Siliciumdioxid oder Aluminosilicat, verbunden ist,
- in Gegenwart eines Säurekatalysators und
- in Gegenwart eines reduzierenden Gases, vorzugsweise Dihydrogen.

2. Verfahren nach Anspruch 1, **dadurch gekennzeichnet, dass** der Metallkatalysator in einer molaren Menge von weniger als 1 %, bezogen auf die molare Menge der Verbindung der allgemeinen Formel II, verwendet wird.

3. Verfahren nach einem der Ansprüche 1 oder 2, **dadurch gekennzeichnet, dass** der Schritt der reduktiven Alkylierung bei einer Temperatur von weniger als 200 °C, vorzugsweise weniger als 180 °C und insbesondere von weniger als 150 °C durchgeführt wird.

4. Verfahren nach einem der Ansprüche 1 bis 3, **dadurch gekennzeichnet, dass** der Säurekatalysator unter den Lewis- oder Brønsted-Säuren ausgewählt wird.

5. Verfahren nach Anspruch 4, **dadurch gekennzeichnet, dass** der Säurekatalysator unter Camphersulfonsäure, para-Toluolsulfonsäure, sauren Sulfonharzen, Trifluoressigsäure, Siliciumdioxid, Siliciumdioxid-Aluminiumoxid, auf Siliciumdioxid getragener Phosphorsäure, BF₃.Et₂O oder Zeolithen ausgewählt wird.

6. Verfahren nach einem der Ansprüche 1 bis 5, **dadurch gekennzeichnet, dass** der Säurekatalysator in einer Menge von weniger als 10 Masse-%, vorzugsweise von weniger als 5 Masse-%, bezogen auf die Gesamtmasse der Verbindung der allgemeinen Formel II, vorhanden ist.

7. Verfahren nach einem der Ansprüche 1 bis 6, **dadurch gekennzeichnet, dass** der Säurekatalysator ein Säurekatalysator ist, der auf festem Träger vorliegt, der mit dem Metallkatalysator ein recycelbares Katalysatorsystem bildet.

8. Verfahren nach einem der Ansprüche 1 bis 7, **dadurch gekennzeichnet, dass** der Säurekatalysator, der auf festem Träger vorliegt, ein saures Sulfonharz ist.

9. Verfahren nach einem der Ansprüche 1 bis 8, **dadurch gekennzeichnet, dass** die Reaktion unter einem Druck von Dihydrogen zwischen 1 und 150 bar, vorzugsweise zwischen 1 und 100 bar, vorzugsweise zwischen 10 und 50 bar, insbesondere zwischen 30 und 50 bar durchgeführt wird.

10. Verfahren nach einem der Ansprüche 1 bis 9, **dadurch gekennzeichnet, dass** in den oben genannten Formeln I, II und III:
- R¹ und R² unabhängig voneinander Gruppen ausgewählt aus einem Wasserstoffatom, einer OH-Gruppe, einer (OCH₂-CH(OH)-CH₂)ₚ-OH-Gruppe mit 1 ≤ p ≤ 10 darstellen,
- R³ eine Gruppe ausgewählt aus einem C₁-C₅₀-Alkyl und einem C₂-C₅₀-Alkenyl darstellt,
- R⁴ eine Gruppe ausgewählt aus einem Wasserstoffatom und einer C₁-C₅-Alkylgruppe darstellt
und wobei mindestens eines von R¹ und R² eine Hydroxylgruppe -OH ist.

11. Verfahren nach einem der Ansprüche 1 bis 10, **dadurch gekennzeichnet, dass** in den oben genannten Formeln I und II:
- R³ eine Gruppe ausgewählt aus einem C₁-C₂₈-Alkyl, vorzugsweise einem C₁-C₂₄-Alkyl, einem C₂-C₂₈-Alkenyl, vorzugsweise einem C₂-C₂₄-Alkenyl, darstellt.

12. Verfahren nach einem der Ansprüche 1 bis 11, **dadurch gekennzeichnet, dass** in den oben genannten Formeln I und III R¹ und R² alle beide eine OH-Gruppe darstellen.

13. Verfahren nach einem der Ansprüche 1 bis 12, **dadurch gekennzeichnet, dass** die Verbindung der allgemeinen Formel II ausgewählt wird aus Valeriansäure, Capronsäure, Adipinsäure, Pelargonsäure, Azelainsäure, Brassylsäure, Myristinsäure, Stearinsäure, Ölsäure, 9Z-Octadecendisäure, Erucasäure und den entsprechenden Methylestern.

## Claims

1. A process for preparing in a single step a polyol ether of formula (I), **characterized in that** it comprises a step of reductive alkylation involving a compound of general formula (II) and a compound of general formula (III): 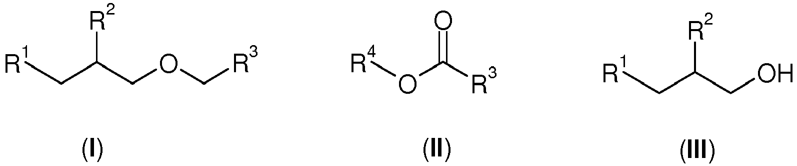 in which:
- R¹ and R² are independently selected from the group consisting of hydrogen atom, an -OH or -(OCH₂-CH(OH)-CH₂)ₚ-OH group with 1≤p≤10;
- R⁴ is an hydrogen atom or is selected from the group consisting of a C₁-C₅₀ alkyl, C₂-C₅₀ alkenyl, aryl, C₃-C₈ cycloalkyl, -CH₂-CH[O-[C(=O)]ₙ-R₅]-CH₂-O-[C(=O)]ₘ-R₆ group, wherein said group may be unsubstituted or substituted;
- R³, R⁵ and R⁶ are independently selected from the group consisting of C₁-C₅₀ alkyl, C₂-C₅₀ alkenyl, aryl, C₃-C₈ cycloalkyl group, wherein said group may be unsubstituted or substituted;
- n and m are independently equal to 0 or 1;
- R⁵ is a hydrogen atom when n=0;
- R⁶ is a hydrogen atom when m=0;
and wherein at least one of R¹ and R² is a hydroxyl group -OH said reductive alkylation step being carried out:
- in the presence of a metal catalyst selected from the group consisting of palladium, rhodium, ruthenium, cobalt, platinum, iridium and nickel, said catalyst optionally being combined with a support selected from the group consisting of charcoal, alumina, silica and aluminosilicate;
- in the presence of an acid catalyst; and
- in the presence of a reductive gas, preferably dihydrogene.

2. The process as claimed in claim 1, wherein said metal catalyst is used in a molar amount of less than 1% relative to the molar amount of the compound of general formula (II).

3. The process as claimed in claim 1 or 2, wherein said reductive alkylation step is carried out at a temperature below 200°C, preferably below 180°C and more preferably below 150°C.

4. The process as claimed in any one of claims 1 to 3, wherein said acid catalyst is selected from the group consisting of Lewis acids and Bronsted acids.

5. The process as claimed in claim 4, wherein said acid catalyst is selected from the group consisting of camphosulfonic acid, paratoluenesulfonic acid, sulfonic acid resins, trifluoroacetic acid, silica, silica-alumina, phosphoric acid supported on silica, BF₃.Et₂O and zeolites.

6. The process as claimed in any one of claims 1 to 5, wherein said acid catalyst is present in an amount of less than 10 weight %, preferably less than 5 weight %, relative to the total weight of the compound of general formula (II).

7. The process as claimed in any one of claims 1 to 6, wherein said acid catalyst is an acid catalyst that is on a solid support, forming, with said metal catalyst, a recyclable catalytic system.

8. The process as claimed in any one of claims 1 to 7, wherein said acid catalyst on a solid support is a sulfonic acid resin.

9. The process as claimed in any one of claims 1 to 8, wherein said reaction is carried out under a pression of dihydrogen comprised between 1 and 150 bars, preferably between 1 and 100 bars, more preferably between 10 and 50 bars, still more preferably between 30 and 50 bars.

10. The process as claimed in any one of claims 1 to 9, wherein in the aforementioned formulae (I), (II) and (III):
- R¹ and R² are independently selected from the group consisting of hydrogen atom, -OH, -(OCH₂-CH(OH)-CH₂)ₚ-OH with 1≤p≤10;
- R³ is selected from C₁-C₅₀ alkyl or C₂-C₅₀ alkenyl;
- R⁴ is selected from hydrogen atom or C₁-C₅ alkyl;
and wherein at least one of R¹ and R² is a hydroxyl group -OH.

11. The process as claimed in any one of claims 1 to 10, wherein in the aforementioned formulae (I) and (II):
- R³ is a group selected from C₁-C₂₈ alkyl, preferably C₁-C₂₄ alkyl, C₂-C₂₈ alkenyl, preferably C₂-C₂₄ alkenyl.

12. The process as claimed in any one of claims 1 to 11, wherein in the aforementioned formulae (I) and (III), R¹ and R² both represent an -OH group.

13. The process as claimed in any one of claims 1 to 12, wherein said compound of general formula (II) is selected from the group consisting of valeric acid, caproic acid, adipic acid, pelargonic acid, azelaic acid, brassylic acid, myristic acid, stearic acid, oleic acid, 9Z-octadecendioic acid, erucic acid and the corresponding methyl esters.
